# FASCICULE DE BREVET EUROPEEN

(11) **EP 1 003 784 B1**
(45) Date de publication et mention de la délivrance du brevet: **07.03.2007**
(21) Numéro de dépôt: 98939690.8
(22) Date de dépôt: 16.07.1998
(51) Int. Cl.: C07K 14/78, C12N 15/63, A61K 38/39

(54) **POLYPEPTIDES UTILES DANS LA REGENERATION DU SYSTEME NERVEUX**
POLYPEPTIDE ZUR REGENERIERUNG DES NERVENSYSTEMS
NOVEL PEPTIDES AND POLYPEPTIDES USEFUL FOR REGENERATING THE NERVOUS SYSTEM

(30) Priorité: 16.07.1997 FR 9709016
(43) Date de publication de la demande: 31.05.2000
(73) Titulaire: Neuronax, 63360 Saint-Beauzire (FR); INSTITUT NATIONAL DE LA SANTE ET DE LA RECHERCHE MEDICALE (INSERM), 75654 Paris Cédex 13 (FR)
(72) Inventeur: MEINIEL, Annie, F-63800 Cournon (FR); MONNERIE, Hubert, F-42300 Roanne (FR); GOBRON, Stéphane, F-63000 Clermont-Ferrand (FR)
(74) Mandataire: Ahner, Francis
(86) Numéro de dépôt international: PCT/FR1998/001556
(87) Numéro de publication internationale: WO 1999/003890

(56) Documents cités:
- EP-A- 0 443 404
- WO-A-93/00430
- WO-A-94/06464
- KLAR E.A.: "F-Spondin: a gene expressed at high levels in the floor plate encodes a secretion protein that promotes neural cell adhesion and neurite extension" CELL, vol. 69, 3 avril 1992, pages 95-110, XP002059005 NA US
- GOBRON E.A.: "SCO-spondin: a new member of the thrombospondin family secreted by the subcommissural organ is a candidate in the modulation of neuronal aggregation" JOURNAL OF CELL SCIENCE, vol. 109, 1996, pages 1053-1061, XP002059006 cité dans la demande
- MOR E.A.: "Induction of neonatal tolerance by plasmid DNA vaccination of mice" J.CLIN.INVEST., vol. 98, no. 12, 15 décembre 1996, pages 2700-2705, XP002084526
- MONNERIE H. ET AL: 'Effect of synthetic peptides derived from SCO-spondin conserved domains on chick cortical and spinal-cord neurons in cell cultures' CELL TISSUE RES vol. 293, 1998, pages 407 - 418
- BURSTYN-COHEN T. ET AL: 'Accumulation of F-Spondin in Injured Peripheral Nerve Promotes the Outgrowth of Sensory Axons' THE JOURNAL OF NEUROSCIENCE vol. 18, no. 21, 01 Novembre 1998, pages 8875 - 8885

### LISTE DE SEQUENCES

(1) INFORMATIONS GENERALES:
   (i) DEPOSANT:
      (A) NOM: UNIVERSITE D'AUVERGNE
      (B) RUE: 49 BOULEVARD FRANCOIS MITTERAND
      (C) VILLE: CLERMONT-FERRAND
      (E) PAYS: FRANCE
      (F) CODE POSTAL: 63000
   (ii) TITRE DE L' INVENTION: NOUVEAUX PEPTIDES ET POLYPEPTIDES UTILES DANS LA REGENERATION DU SYSTEME NERVEUX
   (iii) NOMBRE DE SEQUENCES: 14
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS (D) LOGICIEL: PatentIn Release #1.0, Version #1.30 (OEB)
   (vi) DONNEES DE LA DEMANDE ANTERIEURE:
      (A) NUMERO DE LA DEMANDE: FR 9709016
      (B) DATE DE DEPOT: 16-JUL-1997
(2) INFORMATIONS POUR LA SEQ ID NO: 1:
   (i) CARACTERISTIQUES DE LA SEQUENCE:
      (A) LONGUEUR: 8 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: caractéristique particulière
      (B) EMPLACEMENT:3
      (D) AUTRES INFORMATIONS : Xaa signifie séquences d'acides aminés comportant de 1 à 5 acides aminés.
   (ix) CARACTERISTIQUE:
      (A) NOM/CLE: caractéristique particulière
      (B) EMPLACEMENT:6
      (D) AUTRES INFORMATIONS : Xaa signifie séquences d'acides aminés comportant de 1 à 5 acides aminés.
   (xi) DESCRIPTION DE LA SEQUENCE: SEQ ID NO: 1:
   Trp Ser Xaa Cys Ser Xaa Cys Gly
   1               5
(2) INFORMATIONS POUR LA SEQ ID NO: 2:
   (i) CARACTERISTIQUES DE LA SEQUENCE:
      (A) LONGUEUR: 9 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 2:
   Trp Ser Pro Cys Ser Val Thr Cys Gly
   1               5
(2) INFORMATIONS POUR LA SEQ ID NO: 3:
   (i) CARACTERISTIQUES DE LA SEQUENCE:
      (A) LONGUEUR: 9 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 3:
   Trp Ser Ser Cys Ser Val Thr Cys Gly
   1               5
(2) INFORMATIONS POUR LA SEQ ID NO: 4:
   (i) CARACTERISTIQUES DE LA SEQUENCE:
      (A) LONGUEUR: 9 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 4:
   Trp Ser Gln Cys Ser Val Thr Cys Gly
   1               5
(2) INFORMATIONS POUR LA SEQ ID NO: 5:
   (i) CARACTERISTIQUES DE LA SEQUENCE:
      (A) LONGUEUR: 8 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: caractéristique particulière
      (B) EMPLACEMENT:3
      (D) AUTRES INFORMATIONS : Xaa signifie Pro ou X1-W-X2-X3 ou X1, X2, X3 choisis parmi G,S et C.
   (ix) CARACTERISTIQUE:
      (A) NOM/CLE: caractéristique particulière
      (B) EMPLACEMENT:6
      (D) AUTRES INFORMATIONS : Xaa signifie séquences d'acides aminés comportant de 1 à 5 acides aminés.
   (xi) DESCRIPTION DE LA SEQUENCE: SEQ ID NO: 5:
   Trp Ser Xaa Cys Ser Xaa Cys Gly
   1               5
(2) INFORMATIONS POUR LA SEQ ID NO: 6:
   (i) CARACTERISTIQUES DE LA SEQUENCE:
      (A) LONGUEUR: 8 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: caractéristique particulière
      (B) EMPLACEMENT:3
      (D) AUTRES INFORMATIONS : Xaa signifie X1-W-S-X3 avec X1 et X3 choisis parmi G,S et C
   (ix) CARACTERISTIQUE:
      (A) NOM/CLE: caractéristique particulière
      (B) EMPLACEMENT:6
      (D) AUTRES INFORMATIONS : Xaa signifie séquences d'acides aminés comportant de 1 à 5 acides aminés.
   (xi) DESCRIPTION DE LA SEQUENCE: SEQ ID NO: 6:
   Trp Ser Xaa Cys Ser Xaa Cys Gly
   1               5
(2) INFORMATIONS POUR LA SEQ ID NO: 7:
   (i) CARACTERISTIQUES DE LA SEQUENCE:
      (A) LONGUEUR: 11 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: caractéristique particulière
      (B) EMPLACEMENT:3
      (D) AUTRES INFORMATIONS : Xaa choisi parmi G, S et C
   (ix) CARACTERISTIQUE:
      (A) NOM/CLE: caractéristique particulière
      (B) EMPLACEMENT:6
      (D) AUTRES INFORMATIONS : Xaa choisi parmi G, S et C
   (ix) CARACTERISTIQUE:
      (A) NOM/CLE: caractéristique particulière
      (B) EMPLACEMENT:9
      (D) AUTRES INFORMATIONS : Xaa signifie séquences d'acides aminés comportant de 1 à 5 acides aminés.
   (xi) DESCRIPTION DE LA SEQUENCE: SEQ ID NO: 7:
   Trp Ser Xaa Trp Ser Xaa Cys Ser Xaa Cys Gly
   1               5                   10
(2) INFORMATIONS POUR LA SEQ ID NO: 8:
   (i) CARACTERISTIQUES DE LA SEQUENCE:
      (A) LONGUEUR: 12 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 8:
   Trp Ser Gly Trp Ser Ser Cys Ser Arg Ser Cys Gly
   1               5                   10
(2) INFORMATIONS POUR LA SEQ ID NO: 9:
   (i) CARACTERISTIQUES DE LA SEQUENCE:
      (A) LONGUEUR: 10 acides aminés
      (B) TYPE: acide aminé
      (C) NOMBRE DE BRINS: simple
      (D) CONFIGURATION: linéaire
   (ii) TYPE DE MOLECULE: peptide
   (ix) CARACTERISTIQUE:
      (A) NOM/CLE: caractéristique particulière
      (B) EMPLACEMENT:1
   (ix) CARACTERISTIQUE:
      (A) NOM/CLE: caractéristique particulière
      (B) EMPLACEMENT:4
      (D) AUTRES INFORMATIONS : Xaa signifie séquences d'acides aminés comportant de 1 à 5 acides aminés.
   (ix) CARACTERISTIQUE:
      (A) NOM/CLE: caractéristique particulière
      (B) EMPLACEMENT:7
      (D) AUTRES INFORMATIONS : Xaa signifie séquences d'acides aminés comportant de 1 à 5 acides aminés.
   (ix) CARACTERISTIQUE:
      (A) NOM/CLE: caractéristique particulière
      (B) EMPLACEMENT:10
   (xi) DESCRIPTION DE LA SEQUENCE: SEQ ID NO: 9:
   Xaa Trp Ser Xaa Cys Ser Xaa Cys Gly Xaa
   1               5                   10
(2) INFORMATIONS POUR LA SEQ ID NO: 10:
   (i) CARACTERISTIQUES DE LA SEQUENCE:
      (A) LONGUEUR: 36 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 10:
      TGGWSNGGNT GGWSNWSNTG YWSNMGNWSN TGYGGN 36
(2) INFORMATIONS POUR LA SEQ ID NO: 11:
   (i) CARACTERISTIQUES DE LA SEQUENCE:
      (A) LONGUEUR: 9 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 11:
   Trp Gly Pro Cys Ser Val Ser Cys Gly
   1               5
(2) INFORMATIONS POUR LA SEQ ID NO: 12:
   (i) CARACTERISTIQUES DE LA SEQUENCE:
      (A) LONGUEUR: 8 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 12:
   Asp Cys Lys Asp Gly Ser Asp Glu
   1               5
(2) INFORMATIONS POUR LA SEQ ID NO: 13:
   (i) CARACTERISTIQUES DE LA SEQUENCE:
      (A) LONGUEUR: 4 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 13:
   Arg Lys Ala Arg
   1
(2) INFORMATIONS POUR LA SEQ ID NO: 14:
   (i) CARACTERISTIQUES DE LA SEQUENCE:
      (A) LONGUEUR: 12 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 14:
   Ser Ser Cys Arg Ser Gly Cys Trp Gly Ser Ser Trp
   1               5                   10

## Description

La présente invention concerne notamment de nouveaux peptides et polypeptides utiles notamment à titre de médicaments dans les thérapies impliquant la régénération des cellules du système nerveux, dans le traitement des neuroblastomes, et utiles également comme additifs dans les cultures de cellules nerveuses.

De très nombreuses protéines comportants des motifs répétés que l'on appelle motifs de type 1 des thrombospondines (TSRs) ont été identifiées ces dernières années. On peut dire que ces protéines ont des activités très variées selon le système biologique dans lequel elles interviennent. Citons comme exemples les mieux étudiés et donc les plus connus, les protéines CS (de circumsporozoïte) qui permettent la fixation aux cellules hépatiques de l'agent de la propagation de la malaria, le sporozoïte de plasmodium falciparum (WO 94/06646) et la thrombospondine sécrétée par les plaquettes sanguines qui intervient dans les phénomènes de thrombose et d'angiogénèse (EP 443 404).

En fait, ce motif de type 1 des thrombospodines (TSR) comporte, dans toutes les protéines étudiées jusqu'alors et évoquées précédemment, environ 60 acides aminés (AA) dont un certain nombre comme des cystéines (C), des tryptophanes (W), des sérines (S), des glycines (G), des arginines (R) et des prolines (P) sont très conservés (voir ci-dessous l'alignement de ces AA conservés dans quelques protéines).

Certains peptides synthétiques, déduits de ces motifs TSRs, ont des propriétés biologiques intéressantes. Ainsi, les motifs CSVTCG permettent l'adhésion des sporozoïtes du plasmodium aux cellules hépatiques, les motifs CSVTCG et WXXW permettent l'attachement cellulaire dans d'autres modèles biologiques, BBXB (B étant un acide aminé basique) lie l'héparine et enfin WSXWS lie certains facteurs de croissance.

La F-spondine a été décrite et sa séquence a été alignée avec celle de la thrombospondine dans Klar et al, (1992), Cell, 69, 95-110.

Les caractéristiques générales de la SCO-spondiné sont décrites notamment dans l'article de Monnerie et al. (soumis) et l'article de Gobron et al, (1996), Journal of Cell Science, 109, 1053-1061, 1996. En particulier, l'alignement de la séquence de la SCO-spondine a mis en évidence des homologies avec des protéines telles que la thrombospondine 1 et 2 (Voir séquence, alignement page 1057 de Gobron et al, (1996), J. of Cell Science 109, 1053-1061, incorporée dans la description par référence.).

L'originalité de la présente invention porte sur l'identification et la sélection d'un nouveau peptide actif dans la régénération du système nerveux, dont la séquence dérive de l'un des TSRs de la SCO-spondine.

Plus particulièrement, la présente invention concerne un peptide ou polypeptide ayant la formule :

-W-S-A₁-C-S-A₂-C-G- (SEQ ID n°1)

dans laquelle A₁ et A₂ sont des séquences d'acides aminés consistant en de 1 à 5 acides aminés, à l'exception des peptides ou polypeptides ayant l'une des séquences suivantes

-W-S-P-C-S-V-T-C-G- (SEQ ID n°2)

-W-S-S-C-S-V-T-C-G- (SEQ ID n°3)

-W-S-Q-C-S-V-T-C-G- (SEQ ID n°4)

Il convient de rappeler que dans l'ensemble de la description, on entend désigner par « acide aminé » aussi bien les acides aminés naturels que les acides aminés non naturels. Par « acide aminé naturel » on entend désigner les acides aminés sous forme L que l'on peut trouver dans les protéines naturelles, c'est-à-dire alanine, arginine, asparagine, acide aspartique, cystéine, glutamine, acide glutamique, glycine, histidine, isoleucine, leucine, lysine, méthionine, phénylalanine, proline, sérine, thréonine, tryptophane, tyrosine et valine. Mais, la présente invention concerne également les acides aminés non naturels, c'est-à-dire les acides aminés précédents sous leur forme D, ainsi que les formes homo de certains acides aminés comme l'arginine, la lysine, la phénylalanine et la sérine ou les formes nor de la leucine ou de la valine.

Il est également possible d'envisager l'utilisation d'autres acides aminés comme, par exemple :
Abu : acide alpha-aminobutyrique
Agm : agmatine
Aib : acide alpha-aminoisobutyrique
F-trp : N-formyle-trp
la sarcosine
la statine
l'ornithine
la désaminotyrosine.

La désaminotyrosine est incorporée à l'extrémité N-terminale alors que l'agmatine et la statine sont incorporées à l'extrémité C-terminale de ces peptides.

De façon préférée, les peptides selon la présente invention A₁ est la proline ou X₁-W-X₂-X₃ (SEQ ID n°5), où X₁, X₂, X₃ sont choisis indépendamment l'un des l'autres parmi G, S et C, c'est-à-dire de petits aminoacides.

De façon encore préférée, A₁ est X₁-W-S-X₃ (SEQ ID n°6) et A₂ est choisi parmi RS, VS et VT.

Les raisons de ces choix apparaîtront à la lecture de certains exemples.

De préférence, le polypeptide selon la présente invention a la structure suivante :

-W-S-X₁-W-S-X₂-C-S-A₂-C-G- (SEQ ID n°7)

Le peptide préféré a la structure suivante :

-W-S-G-W-S-S-C-S-R-S-C-G- (SEQ ID n°8)

Enfin, la modification des extrémités peut faciliter l'incorporation de ces produits dans des formes galéniques particulières comme, par exemple, des liposomes ou des microparticules.

La présente invention concerne également des vecteurs d'expression d'ADN caractérisés en ce qu'ils sont capables d'exprimer les peptides ou polypeptides précédents.

Les séquences d'ADN codant pour les peptides ou les polypeptides précédents peuvent être aisément déterminées à partir des séquences en amino-acides ou en s'appuyant, par exemple, sur les séquences naturelles telles qu'elles seront décrites dans la présente demande.

Les vecteurs d'administration pourront être constitués soit par des vecteurs d'ADN nus, soit par des vecteurs plasmidiques, soit encore par des vecteurs viraux ou bien des vecteurs synthétiques.

Il s'agit là de technologies connues qui ne seront pas décrites dans le détail.

L'utilisation de ces vecteurs d'expression permet d'exprimer *in situ* les peptides ou polypeptides en cause et, dans certains cas, est susceptible d'améliorer leur activité.

On choisira, bien entendu, des constructions qui présentent si possible une spécificité pour les cellules nerveuses, dans la mesure où il s'agit des cibles préférées pour les polypeptides selon la présente invention.

Les peptides et polypeptides selon la présente invention peuvent être préparés par tout procédé approprié, notamment ils peuvent être obtenus par synthèse chimique mais également il est possible de les obtenir par voie biologique en utilisant notamment les vecteurs mentionnés précédemment dans les cultures cellulaires appropriées.

Il convient d'ailleurs de remarquer à ce propos que les polypeptides et peptides selon la présente invention peuvent se présenter sous forme déglycosylée ou bien glycosylée si cela est nécessaire. Il convient également de remarquer que dans certains cas et suivant la méthode de préparation il pourra être nécessaire de renaturer certaines structures tertiaires du peptide.

Enfin, les polypeptides selon la présente invention sont plus particulièrement utilisables pour la fabrication d'un médicament et ceci dans le but d'être administrés *in vivo,* notamment dans toutes les pathologies et traumatismes nécessitant une régénération des cellules du système nerveux, et plus particulièrement de leurs prolongements et synapses.

Il peut s'agir de pathologies ou de traumatismes dans lesquels on observe une neurodégénérescence, mais il peut également s'agir de pathologies ou de traumatismes dans lesquels la régénération du système nerveux central, notamment des axones, ou des nerfs périphériques est nécessaire.

Parmi les pathologies neurodégénératives dans lesquelles les composés selon la présente invention peuvent apporter un support, il faut citer notamment la maladie d'Alzheimer, la sclérose en plaques, la maladie de Parkinson et les différents types de myopathies.

Pour ce qui concerne la régénération des prolongements neuronaux, notamment des axones, il peut s'agir en particulier de problèmes de type accidentel ou traumatique (section de moelle épinière ou de nerfs périphériques).

De même, les composés selon la présente invention peuvent être utilisés comme additifs dans certaines cultures cellulaires avec les mêmes effets que ceux mentionnés précédemment sur la croissance des cellules.

Plus particulièrement, les composés selon la présente invention augmentent la pousse neuritique (incluant les axones) dans les neurones du cortex cérébral. Sur les neurones de la moelle épinière on note une inhibition de l'agrégation et une défasciculation des neurites, on note aussi une augmentation des contacts synaptiques.

La « pousse neuritique » est définie comme un allongement, c'est-à-dire une croissance des prolongements des neurones, que ce soit le prolongement dendritique ou axonal.

L'« agrégation » est définie comme un regroupement des cellules formant un amas.

La « défasciculation » est définie comme le résultat d'une diminution de l'adhésivité entre neurites, conduisant à un réseau làche de prolongements neuronaux.

Le « contact synaptique » est défini comme la capacité pour une cellule neuronale de communiquer avec une autre cellule, celle-ci pouvant être également neuronale.

Dans un autre aspect de la présente invention, lesdits peptides ou polypeptides peuvent être utiles pour induire la régression de la tumorogénicité lors de neuroblastome.

La nomenclature utilisée pour décrire la séquence du présent peptide est la nomenclature internationale utilisant le code à trois lettres ou le code à une lettre et où l'extrémité amino-terminale est présentée à gauche et l'extrémité carboxy-terminale est présentée à droite.

Les compositions selon la présente invention peuvent se présenter sous une forme quelconque habituelle pour l'administration pharmaceutique, c'est-à-dire par exemple des formes d'administration liquide en gel ou tout autre support permettant, par exemple, la libération contrôlée.

Parmi les compositions utilisables, il faut citer notamment les compositions injectables plus particulièrement destinées aux injections dans les espaces méningés et sous arachnoïdiens.

### Le peptide le plus actif selon la présente invention a la formule suivante :

Trp-Ser-Gly-Trp-Ser-Ser-Cys-Ser-Arg-Ser-Cys-Gly (SEQ ID n°8)

Il est soluble en milieu aqueux basique, présente un poids moléculaire de 1301 Da et présente une composition en aminoacide de :

| | N | N(%) | P.M. | P.M.(%) |
|---|---|---|---|---|
| C Cys Cystéine | 2 | 16,7 | 206 | 15,8 |
| G Gly Glycine | 2 | 16,7 | 114 | 8,8 |
| R Arg Arginine | 1 | 8,3 | 156 | 12,0 |
| S Ser Serine | 5 | 41,7 | 435 | 33,4 |
| W Trp Tryptophane | 2 | 16,7 | 372 | 28,6 |

Il a été obtenu par synthèse chimique en phase solide.

Mais, comme cela a été indiqué précédemment, il peut être obtenu par génie génétique en utilisant un système hôte-vecteur comprenant l'ADN codant pour le peptide en tenant compte, par exemple, de la dégénérescence afin de le produire en grande quantité.

La séquence d'ADNc codant pour le peptide peut être présentée de la façon suivante (SEQ ID n° 10) :

5'TGG WSN GGN TGG WSN WSN TGY WSN MGN WSN TGY GGN 3'

| | |
|---|---|
| A = Adénosine | W = A ou T |
| C = Cytosine | S = G ou C |
| G = Guanosine | Y = C ou T |
| T = Thymidine | M = A ou C |
| N = A, C, G ou T | |

Le peptide ainsi obtenu a été identifié par microséquençage, analyse HPLC, spectrométrie de masse et séquençage de l'ADN complémentaire.

C'est sur ce peptide (SEQ ID N°8) que l'on a réalisé les expériences décrites ci-après.

### Exemple 1 : effet du peptide SEQ ID n° 8 sur la croissance des neurones

### Matériel et méthode

### Cultures cellulaires dissociées d'hémisphères cérébraux d'embryons de poulet âgés de 8 jours

Les cultures neuronales sont obtenues à partir d'embryons de poulet âgés de 8 jours. Les hémisphères cérébraux, après enlèvement des méninges, sont coupés en petits morceaux et dissociés enzymatiquement avec 0,25 % de trypsine dans un tampon salin PBS exempt de calcium et de magnésium pendant 15 minutes à 37°C.

Les cellules sont centrifugées à 200 g pendant 5 minutes en milieu DMEM à 20 % de SVF pour l'inactivation trypsique. Les cellules sont ensuite filtrées sur membrane de nylon (dimension des pores : 48 microns) et collectées dans un milieu chimiquement défini exempt de sérum contenant un mélange 1/ 1 de DMEM et de milieu Ham's F12 supplémenté avec de la glutamine (4 mM), du glucose (33 mM), de la penicilline G (50 U/ml), du sulfate de streptomycine (50 µg/ml) et un supplément N2 de Bottenstein et Sato (1979) : putrescine (100 µM), sélénite de sodium (30 nM), transferrine humaine (50 µg/ml), progestérone (20 nM), insuline (5 µg /ml) et β-estradiol (1 pM). Tous les suppléments N2 sont achetés chez Sigma.

Les cellules sont étalées avec une densité de 7,5 x 10⁴ cellules/cm² sur des boîtes en plastique à 24 puits. Pour certaines expérimentations, les boîtes en plastique sont revêtues soit de fibronectine (24 µg/ml) soit de thrombospondine (20 µg/ml). Les cultures sont incubées à 37°C et sous air à 10 % de CO₂. Le milieu n'est pas changé au cours de l'expérience. Ces cultures consistent en près de 95 % de neurones.

### Cultures cellulaires de neurones spinales

Les moelles épinières d'embryons de poulet âgés de 6 jours sont disséquées, débarrassées de leur membrane méningée et coupées en petits morceaux dans un tampon phosphate (PBS) exempt de calcium et de magnésium. Après incubation avec 0,25 % de trypsine pendant 10 minutes à 37°C, le tissu est centrifugé à 200 g pendant 4 minutes dans un milieu de croissance contenant 20 % de sérum de veau foetal pour stopper la trypsination. Les cellules sont alors dissociées par trituration répétée en utilisant une pipette Pasteur et resuspendues dans un milieu chimiquement défini exempt de sérum comme précédemment.

Les cellules sont étalées avec une densité de 7,5 x 10⁴ cellules/cm² sur des boîtes de culture en plastique à 24 puits. Les cultures sont incubées à 37°C et sous air à 10 % de CO². Le milieu n'est pas changé durant les expériences et l'on a déjà démontré que ce type de population cellulaire contenait plus de 93 % de neurones.

Les peptides testés sont, outre le peptide selon la présente invention mentionné précédemment (peptide SEQ ID n°8), un second peptide selon l'invention ayant la structure :

W-G-P-C-S-V-S-C-G- (SEQ ID n° 11)

puis 3 peptides de comparaison :

D-C-K-D-G-S-D-E- (SEQ ID n° 12)

R-K-A-R- (SEQ ID n° 13)

et une séquence mélangée du peptide SEQ ID n°8 :

S-S-C-R-S-G-C-W-G-S-S-W- (SEQ ID n° 14).

L'ensemble de ces peptides a été obtenu par synthèse.

### Résultats

En présence du peptide SEQ ID n°8, les neurones s'agrègent et sont essentiellement connectés par des faisceaux de neurites longs et épais après 5 jours de culture. En plus, ces cellules adhèrent bien au substrat revêtu du peptide avec aucun détachement des agrégats. Au contraire, les cultures cellulaires contrôles, en l'absence du peptide, se détachent rapidement du substrat plastique à 5 jours de culture. Toutefois, sur le plastique, seules les neurones corticaux forment des agrégats à partir desquels très peu de neurites peuvent être observées, ce qui indique que le substrat est insuffisamment adhésif. Le nombre des agrégats neuronaux augmente de 9,3 % entre la culture contrôle et la culture traitée avec le peptide selon l'invention.

L'analyse morphométrique révèle un accroissement significatif, à la fois dans le nombre des neurites par agrégat et dans la longueur des neurites par agrégat. D'autre part, des puits de plastique revêtu avec de la BSA sont très peu adhésifs pour les cellules neuronales.

Les essais effectués avec d'autres peptides en comparaison avec le peptide SEQ ID n°8 dans le désordre ne donnent aucun résultat significatif.

Le peptide SEQ ID n°11 donne des résultats plus faibles mais, néanmoins, significatifs.

De même, les essais réalisés avec le peptide SEQ ID n° 13 qui est une séquence consensus de fixation des glycosamino-glycanes présente dans un grand nombre de protéines se fixant à l'héparine, de même que les peptides correspondant à des récepteurs de LDL type A n'ont donné aucun résultat représentatif.

D'autre part, on a étudié l'effet des peptides selon la présente invention SEQ ID n°8 et n°11 sur des cultures à faible densité. En effet, il a déjà été démontré que la forte agrégation pouvait influencer la croissance neuritique de la même façon que la force d'adhésion des cellules au substrat.

Les essais réalisés à faible densité ont montré qu'en l'absence d'agrégation les deux peptides augmentaient de façon significative le pourcentage des cellules neuronales portant des neurites. Dans les contrôles, seuls 24,4 % des cellules adhérantes présentaient des neurites à 4 jours de culture tandis que respectivement 2 et 2,5 fois plus apparaissaient en présence des peptides SEQ ID n°8 et n°11.

Les analyses morphométriques ont révélé une augmentation significative dans chacun d'eux à la fois du nombre de neurites par cellule et de la longueur des neurites en présence du peptide SEQ ID n°8 et non pas du peptide SEQ ID n°11. Dans ces conditions, ceci démontre que, même en l'absence d'agrégation neuronale, le peptide SEQ ID n°8 et à moindre degré le peptide SEQ ID n° 11 sont capables de promouvoir l'adhésion et la croissance neuritique des cellules neuronales corticales.

On a également étudié l'effet du peptide SEQ ID n°8 invention dans différentes conditions expérimentales :

En présence de différents substrats, on a pu démontrer par exemple que le peptide selon l'invention augmentait de façon significative le nombre des neurites par agrégat dans des plaques à puits revêtues avec la thrombospondine ou la fibronectine et ceci par rapport aux contrôles, de même que la longueur des neurites par agrégat.

L'activité du peptide SEQ ID n°8 sur les cultures de cellules de moelle épinière par rapport à des contrôles montre que les neurones demeurent distribués pendant au moins une semaine *in vitro.* Les neurones montrent des croissances neuritiques proéminentes formant un réseau sans fasciculation des neurites. On note une augmentation du nombre des contacts synaptiques entre les neurites. Au contraire, les cellules neuronales des contrôles forment, en général, de petits agrégats interconnectés par de longs filaments. Les neurites croissant à partir des agrégats forment des faisceaux relativement raides le long desquels des neurones essentiellement simples, bi ou tripolaires peuvent être vus.

Les autres peptides testés dans les mêmes conditions ne montrent pas de différence notable par rapport aux contrôles.

### Exemple 2 : Effet du peptide SEQ ID n°8 sur le neuroblastome dérivé de NIB104

### Matériel et méthode

Les cellules dérivées du neuroblastome NIB104 ont été cultivées dans des boîtes plastique 24 puits préalablement recouvertes par un film de polyl-L-ysine, dans des conditions similaires à celles des cultures primaires.

### Résultats

En présence du peptide SEQ ID n°8 selon la présente invention, les cellules de neuroblastomes NIB104 sont nettement moins nombreuses que dans les cultures témoins. L'aspect des cellules est considérablement modifié car elles acquièrent un phénotype neuronal caractéristique. L'analyse morphométrique révèle qu'en présence de concentration croissantes de peptide dans le milieu de culture la pousse neuritique augmente progressivement. Cette réponse est donc dose-dépendant et significative d'un effet physiologique spécifique.

## Revendications

1. Peptide consistant en une séquence répondant à la séquence suivante :
-W-S-A₁-C-S-A₂-C-G- (SEQ ID n°1)
dans laquelle A₁ et A₂ sont des séquences d'acides aminés consistant en de 1 à 5 acides aminés, à l'exception des peptides ayant l'une des séquences suivantes
-W-S-P-C-S-V-T-C-G- (SEQ ID n°2)
-W-S-S-C-S-V-T-C-G- (SEQ ID n°3)
-W-S-Q-C-S-V-T-C-G- (SEQ ID n°4).

2. Peptide selon la revendication 1, **caractérisé en ce que** A₁ est Pro ou -X₁-W-X₂-X₃- (SEQ ID n°5), X₁, X₂ et X₃ étant choisis indépendamment l'un des autres parmi G, S et C.

3. Peptide selon la revendication 2, **caractérisé en ce que** A₁ est X₁-W-S-X₃ (SEQ ID n°6).

4. Peptide selon l'une des revendications 1 à 3, **caractérisé en ce que** A₂ est choisi parmi : R-S, V-S et V-T.

5. Peptide selon l'une des revendications 1 à 4, **caractérisé en ce qu'**il consiste en la séquence -W-S-X₁-W-S-X₂-C-S-A₂-C-G-. (SEQ ID n°7)

6. Peptide selon l'une des revendications 1 à 5, **caractérisé en ce qu'**il s'agit de : -W-S-G-W-S-S-C-S-R-S-C-G- (SEQ ID n°8)

7. Composition pharmaceutique comportant au moins un peptide selon l'une des revendications 1 à 6 et un véhicule pharmaceutiquement acceptable.

8. Utilisation d'un peptide sélectionné parmi les peptides selon l'une des revendications 1 à 6 et les peptides ayant l'une des séquences SEQ ID n°2-4 pour la fabrication d'un médicament destiné à la régénération des cellules du système nerveux.

9. Utilisation d'un peptide sélectionné parmi les peptides selon l'une des revendications 1 à 6 et les peptides ayant l'une des séquences SEQ ID n°2-4 pour la fabrication d'un médicament destiné au traitement des maladies neurodégénératives.

10. Utilisation d'un peptide sélectionné parmi les peptides selon l'une des revendications 1 à 6 et les peptides ayant l'une des séquences SEQ ID n°2-4 pour la fabrication d'un médicament destiné au traitement de pathologies et traumatismes nécessitant une régénération des cellules du système nerveux et plus particulièrement de leur prolongements synaptiques.

11. Utilisation d'un peptide sélectionné parmi les peptides selon l'une des revendications 1 à 6 et les peptides ayant l'une des séquences SEQ ID n°2-4 pour la fabrication d'un médicament destiné au traitement des neuroblastomes.

12. Milieu de culture de cellules nerveuses, **caractérisé en ce qu'**il comporte un peptide sélectionné parmi les peptides selon l'une des revendications 1 à 6.

13. Vecteur d'expression cellulaire **caractérisé en ce qu'**il comporte une séquence d'acide nucléique exprimant un peptide sélectionné parmi les peptides selon l'une des revendications 1 à 6.

14. Vecteur d'expression cellulaire selon la revendication 13 **caractérisé en ce qu'**il comprend une séquence codant pour le peptide de séquence SEQ ID n°8.

## Claims

1. Peptide consisting of a sequence corresponding to the following sequence:
-W-S-A₁-C-S-A₂-C-G- (SEQ ID No. 1)
in which A₁ and A₂ are amino acid sequences consisting of 1 to 5 amino acids with the exception of the peptides having one of the following sequences:
-W-S-P-C-S-V-T-C-G- (SEQ ID No. 2)
-W-S-S-C-S-V-T-C-G- (SEQ ID No. 3)
-W-S-Q-C-S-V-T-C-G- (SEQ ID No. 4).

2. Peptide according to Claim 1, **characterized in that** A₁ is Pro or -X₁-W-X₂-X₃- (SEQ ID No. 5), X₁, X₂ and X₃ being chosen, independently of each other, from G, S and C.

3. Peptide according to Claim 2, **characterized in that** A₁ is X₁-W-S-X₃ (SEQ ID No. 6).

4. Peptide according to one of Claims 1 to 3, **characterized in that** A₂ is chosen from: R-S, V-S and V-T.

5. Peptide according to one of Claims 1 to 4, **characterized in that** it consists of the sequence -W-S-X₁-W-S-X₂C-S-A₂-C-G- (SEQ ID No. 7).

6. Peptide according to one of Claims 1 to 5, **characterized in that** it is: -W-S-G-W-S-S-C-S-R-S-C-G-(SEQ ID No. 8).

7. Pharmaceutical composition comprising at least one peptide according to one of Claims 1 to 6 and a pharmaceutically acceptable vehicle.

8. Use of a peptide selected from the peptides according to one of Claims 1 to 6 and the peptides having one of the sequences SEQ ID Nos. 2-4 for the manufacture of a medicine intended for the regeneration of the nervous system cells.

9. Use of a peptide selected from the peptides according to one of Claims 1 to 6 and the peptides having one of the sequences SEQ ID Nos. 2-4 for the manufacture of a medicine intended for the treatment of neurodegenerative diseases.

10. Use of a peptide selected from the peptides according to one of Claims 1 to 6 and the peptides having one of the sequences SEQ ID Nos. 2-4 for the manufacture of a medicine intended for the treatment of pathological conditions and traumas requiring regeneration of the nervous system cells and more particularly of their synaptic outgrowths.

11. Use of a peptide selected from the peptides according to one of Claims 1 to 6 and the peptides having one of the sequences SEQ ID Nos. 2-4 for the manufacture of a medicine intended for the treatment of neuroblastomas.

12. Culture medium for nerve cells, **characterized in that** it comprises a peptide selected from the peptides according to one of Claims 1 to 6.

13. Cellular expression vector, **characterized in that** it comprises a nucleic acid sequence expressing a peptide selected from the peptides according to one of Claims 1 to 6.

14. Cellular expression vector according to Claim 13, **characterized in that** it comprises a sequence encoding the peptide of sequence SEQ ID No. 8.

## Patentansprüche

1. Peptid, das aus einer Sequenz besteht, welcher der folgenden Sequenz entspricht
-W-S-A₁-C-S-A₂-C-G (SEQ ID NO:1)
in der A₁ und A₂ Aminosäuresequenzen sind, die aus 1 bis 5 Aminosäuren bestehen, mit der Ausnahme der Peptide, die eine der folgenden Sequenzen aufweisen:
-W-S-P-C-S-V-T-C-G (SEQ ID NO:2)
-W-S-S-C-S-V-T-C-G (SEQ ID NO:3)
-W-S-Q-C-S-V-T-C-G (SEQ ID NO:4).

2. Peptid nach Anspruch 1, **dadurch gekennzeichnet, dass** A₁ Pro oder -X₁-W-X₂-X₃- (SEQ ID NO:5) ist, wobei X₁, X₂ und X₃ unabhängig voneinander aus G, S und C ausgewählt sind.

3. Peptid nach Anspruch 2, **dadurch gekennzeichnet, dass** A₁ für X₁-W-S-X₃ (SEQ ID NO:6) steht.

4. Peptid nach einem der Ansprüche 1 bis 3, **dadurch gekennzeichnet, dass** A₂ aus: R-S, V-S und V-T ausgewählt ist.

5. Peptid nach einem der Ansprüche 1 bis 4, **dadurch gekennzeichnet, dass** es aus der Sequenz -W-S-X₁-W-S-X₂-C-S-A₂-C-G (SEQ ID NO:7) besteht.

6. Peptid nach einem der Ansprüche 1 bis 5, **dadurch gekennzeichnet, dass** es sich um: -W-S-G-W-S-S-C-S-R-S-C-G- (SEQ ID NO:8) handelt.

7. Pharmazeutische Zusammensetzung, umfassend mindestens ein Peptid nach einem der Ansprüche 1 bis 6 und ein pharmazeutisch annehmbares Vehikel.

8. Verwendung eines Peptids, das aus den Peptiden nach einem der Ansprüche 1 bis 6 und den Peptiden mit einer der Sequenzen SEQ ID NO:2-4 ausgewählt ist, für die Herstellung eines Medikaments, das für die Regeneration von Zellen des Nervensystems bestimmt ist.

9. Verwendung eines Peptids, das aus den Peptiden nach einem der Ansprüche 1 bis 6 und den Peptiden mit einer der Sequenzen SEQ ID NO:2-4 ausgewählt ist, für die Herstellung eines Medikaments, das zur Behandlung von neurodegenerativen Krankheiten bestimmt ist.

10. Verwendung eines Peptids, das aus den Peptiden nach einem der Ansprüche 1 bis 6 und den Peptiden mit einer der Sequenzen SEQ ID NO:2-4 ausgewählt ist, für die Herstellung eines Medikaments, das zur Behandlung von Krankheiten und Traumen bestimmt ist, die eine Regeneration von Zellen des Nervensystems und spezieller ihrer synaptischen Fortsätze erfordert.

11. Verwendung eines Peptids, das aus den Peptiden nach einem der Ansprüche 1 bis 6 und den Peptiden mit einer der Sequenzen SEQ ID NO:2-4 ausgewählt ist, für die Herstellung eines Medikaments, das zur Behandlung von Neuroblastomen bestimmt ist.

12. Nervenzellen-Kulturmedium, **dadurch gekennzeichnet, dass** es ein Peptid enthält, das aus den Peptiden nach einem der Ansprüche 1 bis 6 ausgewählt ist.

13. Zellulärer Expressionsvektor, **dadurch gekennzeichnet, dass** er eine Nukleinsäuresequenz enthält, die ein Peptid exprimiert, das aus den Peptiden nach einem der Ansprüche 1 bis 6 ausgewählt ist.

14. Zellulärer Expressionsvektor nach Anspruch 13, **dadurch gekennzeichnet, dass** er eine Sequenz enthält, die für das Peptid der Sequenz SEQ ID NO:8 kodiert.
